# EUROPEAN PATENT APPLICATION

(11) **EP 4 470 594 A1**
(43) Date of publication of application: **04.12.2024**
(21) Application number: 23747087.7
(22) Date of filing: 27.01.2023
(51) Int. Cl.: A61M 37/00

(54) **APPLICATOR, AND MICRONEEDLE PATCH KIT**

(30) Priority: 28.01.2022 JP 2022011472
(71) Applicant: MEDRx Co., Ltd., Higashikagawa-shi Kagawa 769-2712 (JP)
(72) Inventor: NAKAMURA, Jun, Higashikagawa-shi, Kagawa 769-2712 (JP); KOBAYASHI, Katsunori, Higashikagawa-shi, Kagawa 769-2712 (JP); HAMAMOTO, Hidetoshi, Higashikagawa-shi, Kagawa 769-2712 (JP)
(74) Representative: Zacco Sweden AB
(86) International application number: PCT/JP2023/002632
(87) International publication number: WO 2023/145877

(57) **Abstract**

An applicator includes a holder 22 configured to hold a microneedle base on which a microneedle array is provided, a pusher 21 for pushing the holder 22 toward skin, and a shell 23 that includes a first part to contact with the skin and a second part configured to hold the holder 22, the shell 23 being deformable while maintaining an orientation of the microneedle base by connecting the first part and the second part.

## Description

### [CROSS-REFERENCE TO RELATED APPLICATION]

The present application is based upon and claims the benefit of priority from Japanese Patent Application No. 2022-011472 filed on January 28, 2022, the entire contents of which are incorporated herein by reference.

### [Technical Field]

The present disclosure relates to an applicator for puncturing skin with a microneedle array and to a microneedle patch kit that includes the applicator.

### [Background Art]

In recent years, as one of transdermal drug administration systems, a drug administration system using a microneedle array has been attracting attention. With fine needles a few hundred microns long being formed at high density on backing material, the microneedle array sticks skin with needles carrying a targeted drug (molecules of a vaccine, protein, peptide, or the like) on a surface or inside and directly transfer the drug to the dermis or the epidermis. Medicine administration using a microneedle array has many advantages including the following: the liver is not burdened unlike when oral medicine is used, there is no pain accompanying insertion of a needle unlike in the case of an injectable drug, and side effects of temporary excessive drug absorption can be alleviated.

A microneedle array described in Patent Literature 1 listed below is aimed at uniformly enlarging wrinkles on skin and intended to effectively stick microneedles into the skin roughened by wrinkles.

### [Citation List]

### [Patent Literature]

[Patent Literature 1]
Japanese Patent No. 6546091

### [Summary of Invention]

Patent Literature 1, which is aimed at effectively sticking microneedles into wrinkled skin, does not disclose how to reliably stick the skin with a large number of microneedles provided in the microneedle array, regardless of the presence or absence of wrinkles.

An object of the present disclosure is to provide an applicator that makes it possible to reliably stick skin with a large number of microneedles provided in a microneedle array, and a microneedle patch kit that includes the applicator.

The present disclosure provides an applicator for puncturing skin with a microneedle array, the applicator including a holder configured to hold a microneedle base on which the microneedle array is provided, a pusher configured to push the holder toward the skin, and a shell that includes a first part to contact with the skin and a second part configured to hold the holder, the shell being deformable while maintaining an orientation of the microneedle base by connecting the first part and the second part.

The present disclosure provides a microneedle patch kit including the applicator, the microneedle base, an adhesive layer formed on a flange of the shell, and a protective sheet configured to close an opening in the shell by abutting the adhesive layer.

### [Brief Description of Drawings]

[Figure 1] Figure 1 is an external perspective view showing the appearance of a microneedle patch kit according to the present embodiment.
[Figure 2] Figure 2 is an exploded perspective view showing a configuration of the microneedle patch kit according to the present embodiment.
[Figure 3] Figure 3 is a diagram of a pusher viewed from above.
[Figure 4] Figure 4 is a partial sectional view showing a section taken along line IV-IV in Figure 3.
[Figure 5] Figure 5 is a partial sectional view showing a section taken along line V-V in Figure 3.
[Figure 6] Figure 6 is a partial sectional view magnifying part VI in Figure 5.
[Figure 7] Figure 7 is a perspective view of the pusher and a holder cut at an upper end of the holder and viewed from above.
[Figure 8] Figure 8 is a partial sectional view of the pusher and the holder.
[Figure 9] Figure 9 is a partial sectional view showing the pusher, the holder, a microneedle base, and a shell.
[Figure 10] Figure 10 is a diagram showing return parts provided on the holder.
[Figure 11] Figure 11 is a partial sectional view showing the holder and the microneedle base.
[Figure 12] Figures 12(A) and 12(B) are partial sectional views showing the holder and the microneedle base.
[Figure 13] Figure 13 is a diagram showing the holder and the microneedle base.
[Figure 14] Figure 14 is a partial sectional view of the shell.
[Figure 15] Figure 15 is a partial sectional view of the shell.
[Figure 16] Figure 16 is a front view of the microneedle base.
[Figure 17] Figure 17 is a right side view of the microneedle base.
[Figure 18] Figure 18 is a plan view of the microneedle base.
[Figure 19] Figure 19 is a bottom view of the microneedle base.
[Figure 20] Figure 20 is a sectional view taken along line XX-XX in Figure 18.
[Figure 21] Figure 21 is a front view of a microneedle patch.
[Figure 22] Figure 22 is a right side view of the microneedle patch.
[Figure 23] Figure 23 is plan view of the microneedle patch.
[Figure 24] Figure 24 is a bottom view of the microneedle patch.
[Figure 25] Figure 25 is a perspective view showing a state in which the microneedle patch is attached to an arm.
[Figure 26] Figure 26 is a perspective view showing a pusher and a holder according to a variation.
[Figure 27] Figure 27 is a sectional view showing a section taken along line XXVII-XXVII in Figure 26.
[Figure 28] Figure 28 is a sectional view showing a section taken along line XXVIII-XXVIII in Figure 26.

### [Description of Embodiments]

The present embodiment will be described below with reference to the accompanying drawings. To facilitate understanding of the description, the same components in different drawings are denoted by the same reference signs whenever possible and redundant description thereof will be omitted.

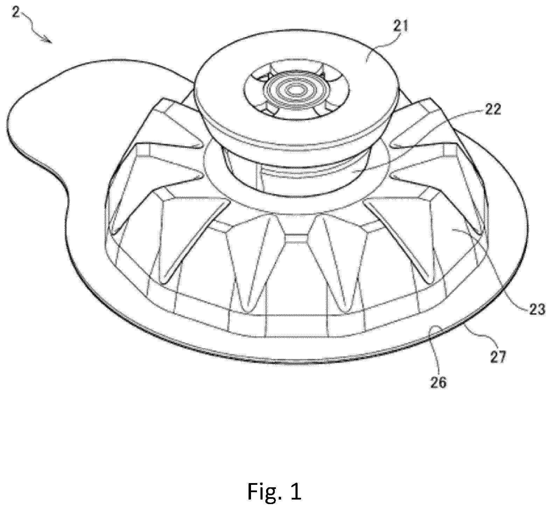

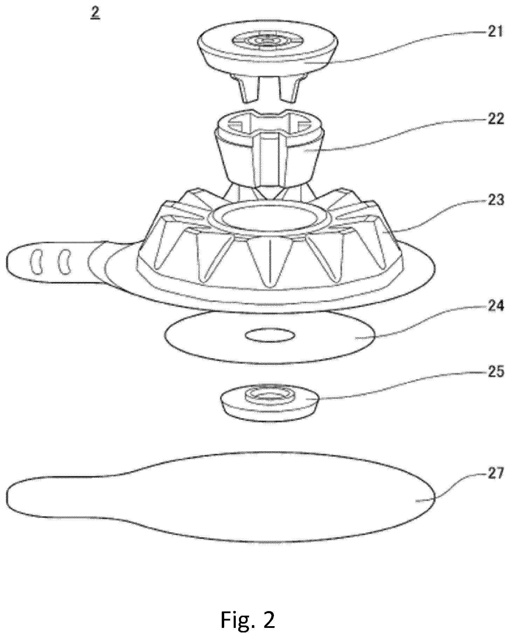

An applicator and microneedle patch kit according to embodiment will be described with reference to Figures 1 and 2. Figure 1 is an external perspective view showing the appearance of the microneedle patch kit 2. Figure 2 is an exploded perspective view showing components of the microneedle patch kit 2.

As shown in Figure 2, the microneedle patch kit 2 includes a pusher 21, a holder 22, a shell 23, a sticky tape 24, a microneedle base 25, an adhesive layer 26, and a protective sheet. An assembly process of the microneedle patch kit 2 involves fitting the pusher 21 over the holder 22. Next, the integrated pusher 21 and holder 22 are fitted into the shell 23. Next, the microneedle base 25 with a microneedle array provided thereon is affixed to the sticky tape 24. Next, the microneedle base 25 with the sticky tape 24 affixed thereto is hooked to a hook of the holder 22. Next, an adhesive is applied to a flange of the shell 23, thereby forming the adhesive layer 26 (See Figure 1). Next, the protective sheet 27 is affixed to the adhesive layer 26. Any excess part is removed from the protective sheet 27 by punching using a die. By going through such a process, the microneedle patch kit 2 such as shown in Figure 1 is completed. Note that the assembly process of the microneedle patch kit 2 is not limited to the above procedures. For example, the assembly process may be performed in the following order: the integrated pusher 21 and holder 22 are fitted into the shell 23, the adhesive layer 26 is formed on the flange of the shell and then the microneedle base 25 with the sticky tape 24 affixed thereto is mounted by being hooked to the hook of the holder 22.

Regarding the adhesive, a publicly-known adhesive can be used. The adhesive layer 26 can be formed using for example, a polymer such as an acrylic polymer, a rubber polymer (e.g., styrene-isoprene-styrene block copolymer, styrene-butadiene-styrene block copolymer, polyisoprene, polyisobutylene, or polybutadiene), a silicone polymer, or a vinyl ether polymer with the polymer being hot-melted (hot-melt process), dissolved in an organic solvent (e.g., ethyl acetate or heptane) (solvent process), or applied.

In the following description, the side on which the pusher 21 of the microneedle patch kit 2 is located is designated as the upper side and the side on which the protective sheet 27 is located is designated as the lower side. The designations of the upper side and lower side do not necessarily correspond to the direction of gravity, and are used for convenience of explanation.

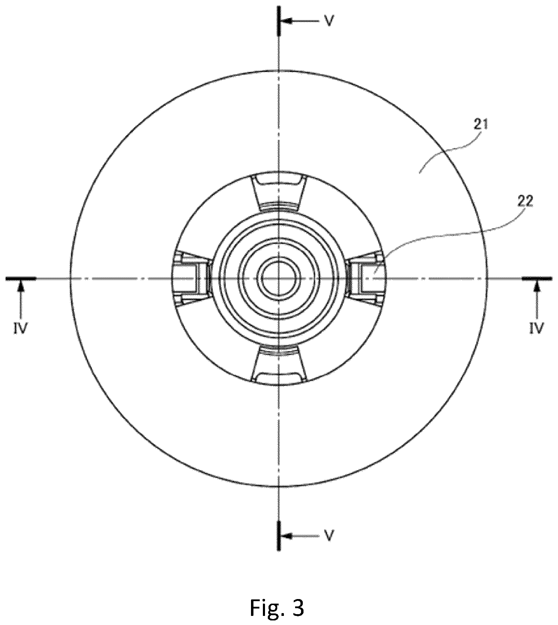

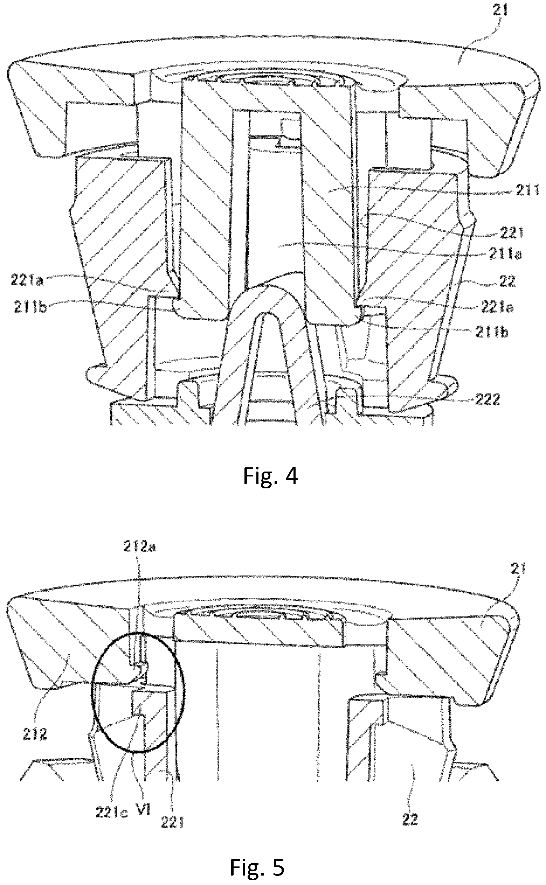

Next, details of components will be described. Figure 3 is a diagram of the pusher 21 viewed from above. Figure 4 is a partial sectional view showing a section taken along line IV-IV in Figure 3. Figure 5 is a partial sectional view showing a section taken along line V-V in Figure 3.

As shown in Figure 4, the holder 22 includes a central recess 221 and a hook portion 222. The central recess 221 includes a temporary holding protrusion 221a. A central projection 211 of the pusher 21 is inserted in the central recess 221 from above. The central projection 211 includes a pushing groove 211a and a temporary holding protrusion 211b. The temporary holding protrusion 211b of the central projection 211 and the temporary holding protrusion 221a of the central recess 221 are fitted together. The pushing groove 211a is abutted against the hook portion 222. In a direction in which the pusher 21 and the holder 22 are separated from each other, the temporary holding protrusion 211b and the temporary holding protrusion 221a are fitted together, thereby restricting movements. In a direction in which the pusher 21 is pushed into the holder 22, the pushing groove 211a abuts the hook portion 222, thereby restricting movements.

As shown in Figure 5, indicator protrusions 221c are provided on an upper end of the central recess 221 of the holder 22. The indicator protrusions 221c are provided protruding radially outward from the central recess 221. Indicator protrusions 212a are provided on a button head 212 of the pusher 21. The indicator protrusions 212a are provided at positions corresponding to the indicator protrusions 221c, protruding radially inward. When a user depresses the pusher 21, the indicator protrusions 212a run upon the indicator protrusions 221c, eventually fitting under the indicator protrusions 221c. In so doing, a notification sound is generated, notifying the user that puncture stress has reached a level high enough to perform puncture. By hearing the notification sound, the user can make sure that the puncture is complete.

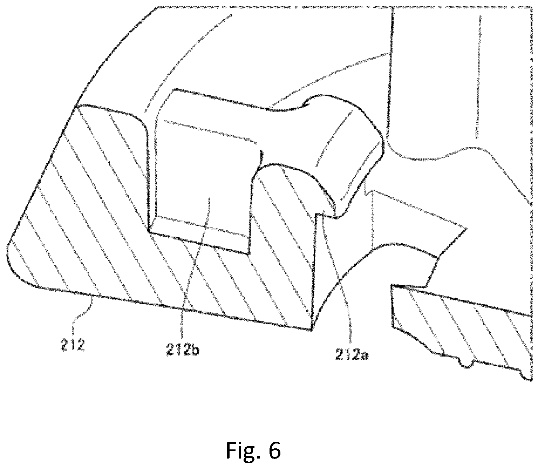

Figure 6 shows part VI in Figure 5 magnified and viewed from below. The button head 212 includes ribs 212b provided at positions corresponding to the indicator protrusions 212a. The ribs 212b allow targeted shear stress to be generated without the indicator protrusions 212a deflecting outward even if abutted against the indicator protrusions 221c.

A plurality of sets of the indicator protrusion 212a and indicator protrusion 221c are provided in a circumferential direction. Because the indicator protrusions 212a and the indicator protrusions 221c are used to generate the notification sound, the notification sounds generated respectively by the multiple sets have to be synchronized. According to the present embodiment, lags in generation of the notification sounds are reduced by reducing wobble produced when the pusher 21 is fitted over the holder 22.

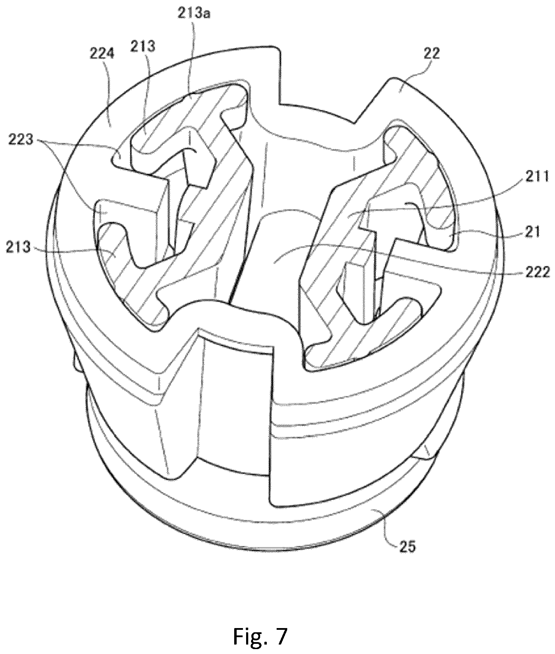

Figure 7 is a diagram of the pusher 21 and holder 22 assembled together and viewed from above by cutting the pusher 21 at an upper end of the holder 22. The holder 22 includes an outer wall 224. Orientation control recesses 223 are provided inside the outer wall 224. Orientation control walls 213 extend from the central projection 211 of the pusher 21 toward the respective orientation control recesses 223. The orientation control walls 213 include respective orientation control protrusions 213a.

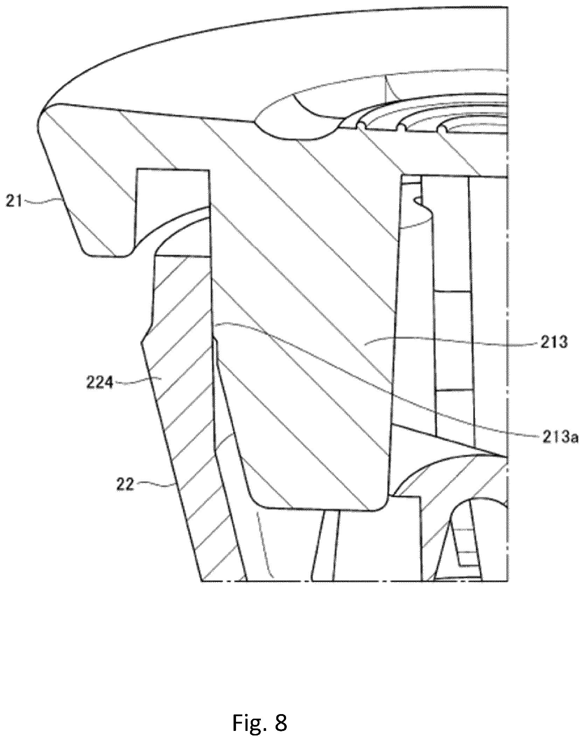

Figure 8 is a partial sectional view for illustrating a relationship between the outer wall 224 and the orientation control walls 213. The orientation control protrusions 213a provided on the orientation control walls 213 are abutted against the inner side of the outer wall 224. Thus, the pusher 21 and the holder 22 are fitted together without wobble, reducing lags in generation of the notification sounds.

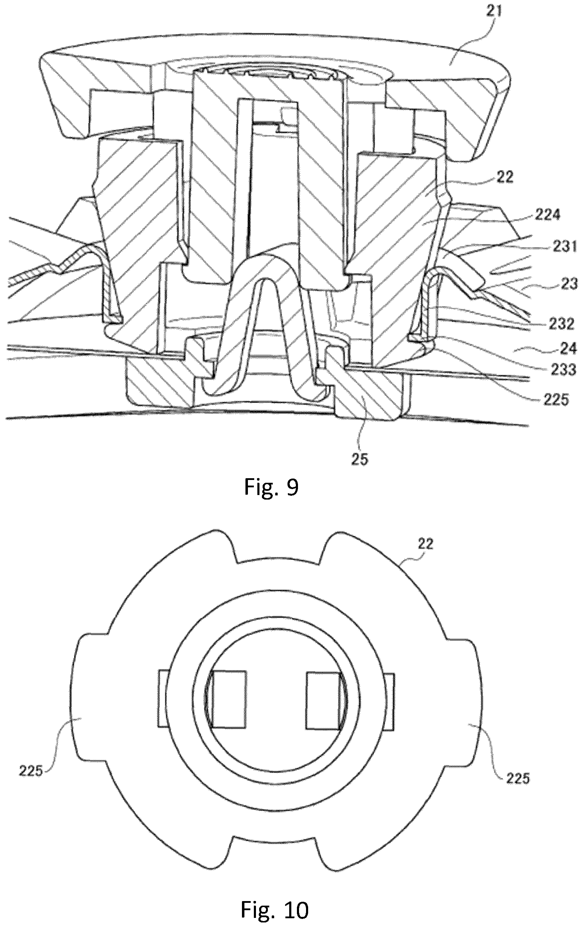

Figure 9 is a diagram for illustrating how the holder 22 is mounted on the shell 23. A through-hole 231 is provided in the shell 23. The shell 23 includes a side wall 232 that forms the through-hole 231. A bend 233 is provide at a lower end of the side wall 232, heading toward a center side of the through-hole 231.

The outer wall 224 of the holder 22 includes return parts 225. The return parts 225 are provided at a lower end of the outer wall 224. The return parts 225 project toward the side wall 232. The outer wall 224 that functions as a rising part rises from base ends of the return parts 225 toward an inner edge of the through-hole 231 by expanding in diameter. Figure 10 is a diagram for illustrating an arrangement mode of the return parts 225. As shown in Figure 10, the return parts 225 are provided at two locations on an outer circumference of the holder 22 so as to be symmetrical to each other with respect to the center of the holder 22. The shape and number of the return parts 225 are not specifically limited, but can be selected, for example, such that the bend 233 will not be deformed when the return parts 225 pass through the bend 233 during assembly. As shown in Figure 10, undersurfaces of the return parts 225 may be sloped to make it easy for the bend 233 to pass.

As the bend 233 gets engaged with the return parts 225, even if only the pusher 21 or the holder 22 is gripped, the microneedle patch kit can be picked up without dropping the shell 23 and lower parts. With this configuration, i.e., when the skin is punctured with the microneedle array and the microneedle array is fixed to the skin by a sticky tape after use the microneedle array can be discarded as an integral part, without the pusher 21 and the holder 22 falling off the shell 23.

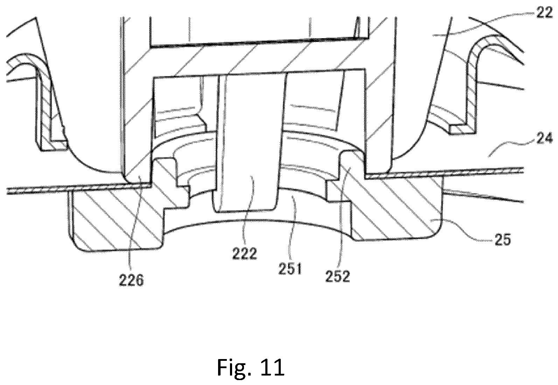

Figure 11 is a diagram for illustrating how the holder 22 holds the microneedle base 25. As shown in Figure 11, the microneedle base 25 includes an opening 251. Although details will be described later, the hook portion 222 is placed extending outward from inside the opening 251. The holder 22 and the microneedle base 25 are shaped such that the microneedle base 25 will not come off easily as follows: when the holder 22 is pushed onto the microneedle base 25, the hook portion 222 easily climbs over the opening 251 in the microneedle base 25 and gets hooked to the opening 251 in the microneedle base 25, holding the microneedle base 25, thereby shaped to prevent easy detachment.

A recess 226 is provided at a lower end of the holder 22. A projection 252 is provided surrounding the opening 251 in the microneedle base 25. As the projection 252 enters the recess 226, the microneedle base 25 is positioned with respect to the holder 22.

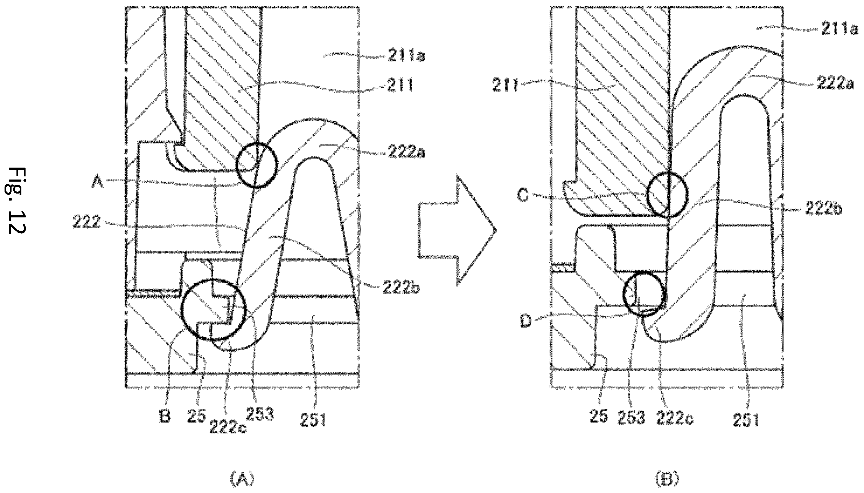

Figures 12(A) and 12(B) are diagrams for illustrating how the hook portion 222 holds and then releases the microneedle base 25. As shown in Figure 12(A), the hook portion 222 includes a base end portion 222a, arms 222b, and engaging parts 222c.

The base end portion 222a, which is provided on the side of the pushing groove 211a, is a part that connects a pair of the arms 222b. The arms 222b are formed to swing around the base end portion 222a under an external force by being pressed by the central projection 211. The engaging parts 222c are provided on one end of the respective arms 222b. The other end of each arm 222b is connected to the base end portion 222a.

The microneedle base 25 includes an annular catcher 253. The annular catcher 253 is formed surrounding the opening 251. As shown in region B, before use, the engaging parts 222c are engaged with the annular catcher 253, keeping the microneedle base 25 held by the holder 22. In the state shown in Figure 12(A), as shown in region A, the arms 222b remain abutted by the central projection 211 without application of force.

As shown in Figure 12(B), when the pusher 21 is depressed, the central projection 211 descends. As shown in region C, the central projection 211 pushes down the arms 222b. The arms 222b swing around the base end portion 222a, reducing a distance between the pair of arms 222b. As shown in region D, the engaging parts 222c are separated from the annular catcher 253. The microneedle base 25 is pushed further toward the skin by the central projection 211 and separated from the holder 22, causing the skin to be punctured with needles of the microneedle array provided on an undersurface of the microneedle base 25, and then fixed to the skin by the sticky tape 24.

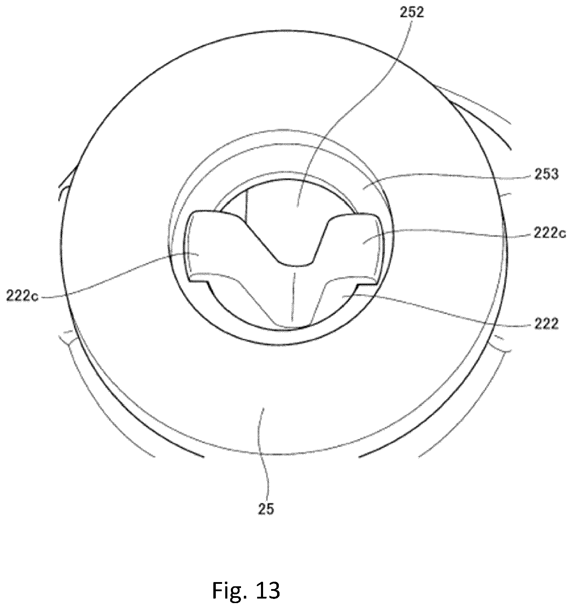

Figure 13 is a perspective view of the microneedle base 25 and hook portion 222 before use as viewed from below. As shown in Figure 13, the engaging parts 222c are formed into an arc shape along the annular catcher 253. By forming the engaging parts 222c into an arc shape, it is possible to make engagement of the engaging parts 222c with the annular catcher 253 constant.

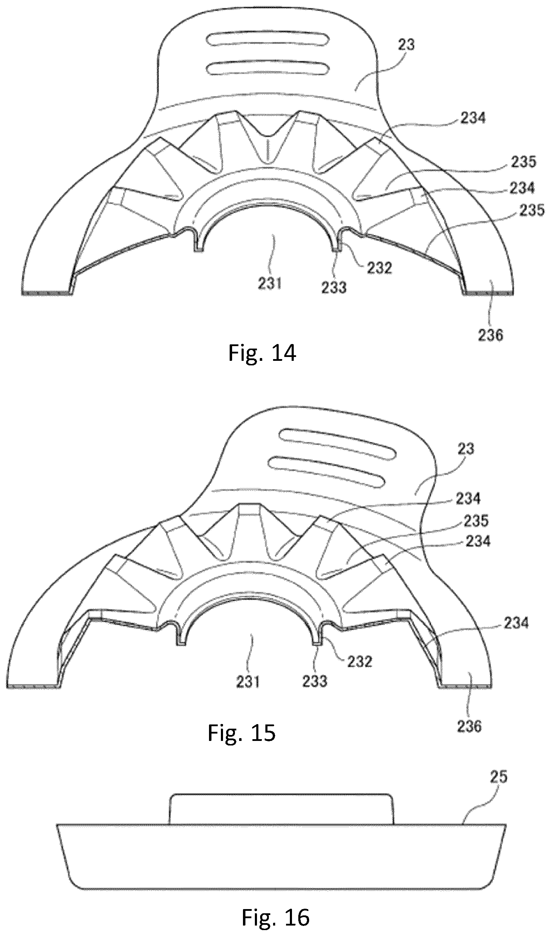

Figures 14 and 15 are partial sectional views for illustrating a shape of the shell 23. The shell 23 includes the side wall 232, the bend 233, ridges 234, valleys 235, and a flange 236. The flange 236 is a part configured to abut the skin. The ridges 234 and the valleys 235 are arranged radially outward from the through-hole 231. The ridges 234 and the valleys 235 are provided alternately in a circumferential direction. Figure 14 shows a section taken along valleys 235. Figure 15 shows a section taken along ridges 234.

As described above, the ridges 234 and valleys 235 configured to deflect while maintaining a posture of the microneedle base when the pusher 21 pushes the holder 22 toward the skin are radially provided alternately on the shell 23. In the present specification, maintaining the posture of the microneedle base 25 means maintaining a state in which a base plate of the microneedle array is substantially parallel to the skin.

Due to the morphological innovation of the shell 23 providing the ridges 234 and valleys 235 alternately and radially, since the holder 22 can be pushed onto the microneedle base 25 while maintaining an orientation of the microneedle base, the skin can be punctured with the microneedles provided on the microneedle base 25 without separately providing orientation stabilization means.

As described with reference to Figures 1 and 2, the microneedle patch kit 2 includes the pusher 21, the holder 22, the shell 23, the sticky tape 24, the microneedle base 25, the adhesive layer 26, and the protective sheet 27. According to the present embodiment, the applicator may be such as to include at least the pusher 21, the holder 22, the shell 23, and the microneedle base 25.

The applicator according to the present embodiment, the microneedle patch kit, is an applicator for puncturing skin with a microneedle array, the applicator including: the holder 22 configured to hold the microneedle base 25 on which the microneedle array is provided; the pusher 21 configured to push the holder 22 toward the skin; and a shell 23 that includes the flange 236 serving as a first part to contact with the skin and the bend 233 serving as a second part configured to hold the holder 22, the shell 23 being deformable while maintaining an orientation of the microneedle base 25 by connecting the first part and the second part.

When an external force is applied to the pusher 21, since the shell 23 deforms while maintaining the orientation of the microneedle base 25, the skin can be punctured by reliably pushing the microneedle base 25 held by the holder 22, against the skin.

According to the present embodiment, the pusher 21 is provided so as to be slidable relative to the holder 22, the holder 22 includes the hook portion 222 configured to hold the microneedle base 25, and when the pusher 21 slides relative to the holder 22, the hook portion 222 releases the microneedle base 25.

For example, when the user applies force to the pusher 21, the holder 22 and the pusher 21 slide relative to each other, allowing the microneedle base 25 to be released from the holder 22 while puncturing the skin with the microneedle array.

According to the present embodiment, the hook portion 222 deforms under an external force applied via the pusher 21, and thereby releases the microneedle base 25. The user can also see whether the puncture stress has reached a required level based on whether the microneedle base 25 is released.

By releasing the microneedle base 25 by such simple means as deformation, it is possible to provide an applicator and microneedle patch kit capable of reliably separating the microneedle patch from the applicator using a simple configuration. From the viewpoint of separating a microneedle patch from an applicator, there has been no applicator capable of reliable separation with a simple configuration. Besides, the present embodiment involves a small number of parts and thereby makes assembly easier.

According to the present embodiment, the opening 251 is provided in the microneedle base 25, and the hook portion 222 is placed extending outward from inside the opening 251, thereby holding the microneedle base 25.

Since the hook portion 222 is placed extending outward from inside the opening 251, the hook portion 222 can be configured to release the microneedle base 25 by deforming inward under an external force applied to the hook portion 222 via the pusher 21, and thus can be made more compact in a radial direction than when the hook portion 222 is deformed outward.

According to the present embodiment, as the projection 252 provided on the microneedle base 25 enters the recess 226 provided in the holder 22, the microneedle base 25 is positioned with respect to the holder 22. The opening 251 is provided inside the projection 252.

Since the recess 226 provided in the holder 22 and the projection 252 provided on the microneedle base 25 are positioned with respect to each other by being fitted together, an appropriate clearance can be provided such that the microneedle base 25 will be held to the holder 22 without wobble, thereby making available such holding power that will not cause obstruction in separating the microneedle base 25. Since the opening 251 is provided inside the projection 252 of the microneedle base 25 and the hook portion 222 is provided in the holder 22, extending outward from the opening 251, i.e., toward the recess 226 from the projection 252, the microneedle base 25 can be held to the holder 22 with appropriate strength without wobble and without standing in the way of separating the microneedle base 25.

The present embodiment also includes the indicator protrusions 212a and 221c serving as indicators configured to slide the pusher 21 relative to the holder 22 and generate notification sounds when a pushing force toward the skin exceeds a predetermined value.

By generating notification sounds when the pushing force toward the skin exceeds a predetermined value, it is possible to determine whether an appropriate force has been applied to the microneedle base 25.

According to the present embodiment, the indicator protrusions 212a and 221c serving as indicators generate notification sounds through rubbing between the holder 22 and the pusher 21.

Since the notification sounds are generated through rubbing between the holder 22 and the pusher 21, the notification sounds can be generated without providing separate sound generating means independently.

According to the present embodiment, a plurality of the indicator protrusions 212a and 221c serving as indicators are provided.

Since the plurality of indicators that generate notification sounds through rubbing between the holder 22 and the pusher 21 are provided, the holder 22 and the pusher 21 each have a plurality of parts that rub against each other, which stabilizes sliding of the pusher 21 along the holder 22.

According to the present embodiment, when the pusher 21 slides relative to the holder 22, the notification sounds generated by the plurality of indicators are synchronized.

As the notification sounds generated at the plurality of locations are synchronized, the user is relieved from wondering whether an appropriate force has been applied. Since the notification sounds are generated through rubbing between the holder 22 and the pusher 21, by synchronizing the generation of the notification sounds the sliding of the pusher 21 along the holder 22 can be stabilized as well.

According to the present embodiment, as the holder 22 is passed through the through-hole 231 provided in the shell 23, the shell 23 holds the holder 22. The shell 23 includes the side wall 232 forming the through-hole 231 and the holder 22 includes the return parts 225 protruding toward the side wall 232.

When the holder 22 is passed through the through-hole 231, the return parts 225 provided in the holder 22 deform the side wall 232 outward, and when the return parts 225 pass through the side wall 232, the side wall 232 returns to its original shape, and thus the holder 22 can be held to the shell 23 by the side wall 232 and the return parts 225.

According to the present embodiment, the side wall 232 includes the bend 233 that conforms in shape to the return parts 225, and the holder 22 includes the outer wall 224 serving as a rising part that rises from the base ends of the return parts 225 toward the inner edge of the through-hole 231 by expanding in diameter.

Since the bend 233 and the return parts 225 abut each other, the holder 22 can be held to the shell 23 more stably. Furthermore, since the outer wall 224 serving as a rising part is provided, rising from the base ends of the return parts 225 toward the inner edge of the through-hole 231 by expanding in diameter, the shell 23 and the outer wall 224 serving as a rising part abut each other in a position away from the return parts 225 and the bend 233, and consequently the holder 22 can be held to the shell 23 more stably.

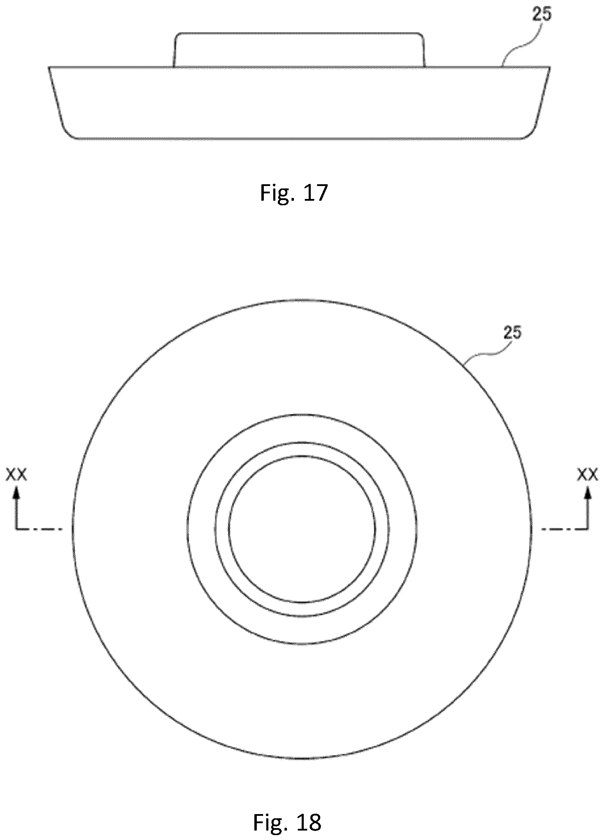

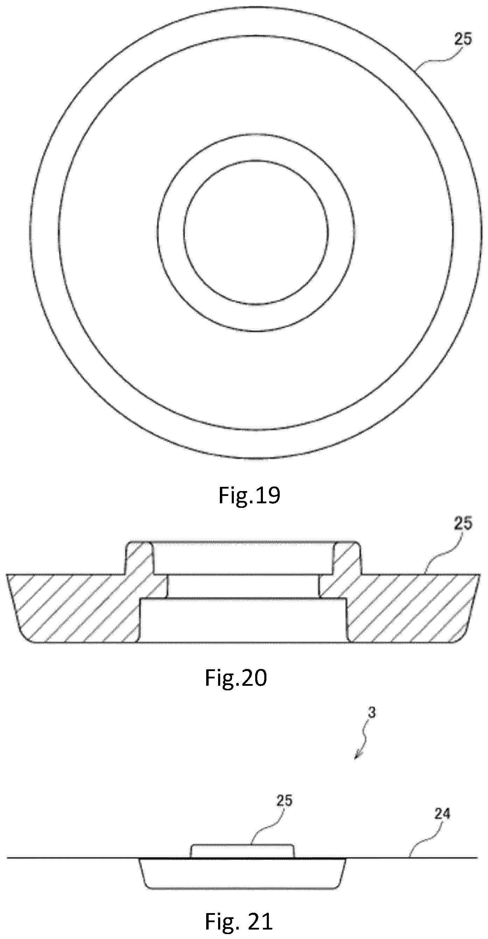

Figure 16 is a front view of the microneedle base 25. Figure 17 is a right side view of the microneedle base 25. Figure 18 is a plan view of the microneedle base 25. Figure 19 is a bottom view of the microneedle base 25. Figure 20 is a sectional view taken along line XX-XX in Figure 18.

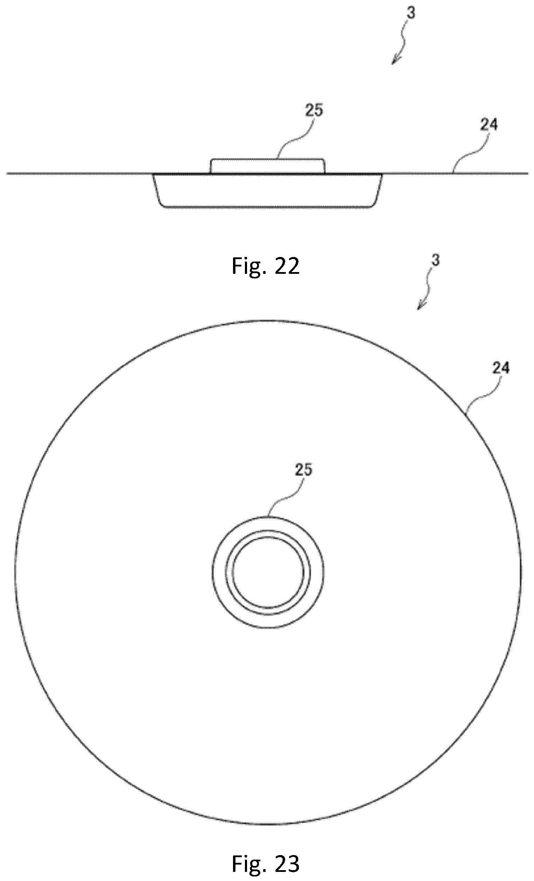

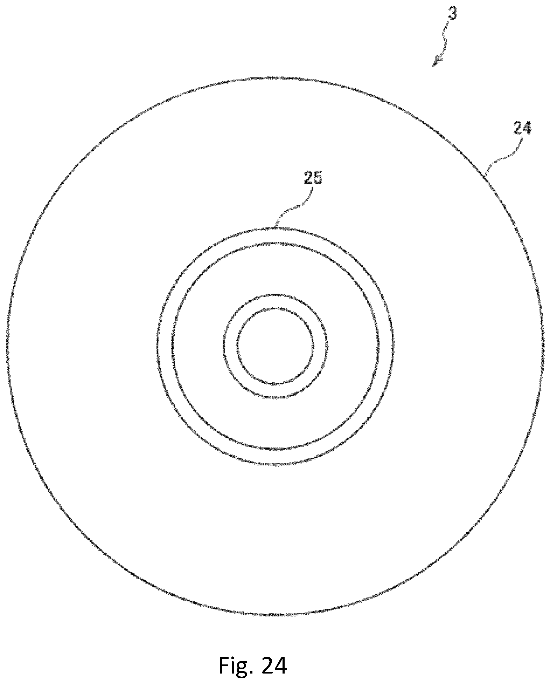

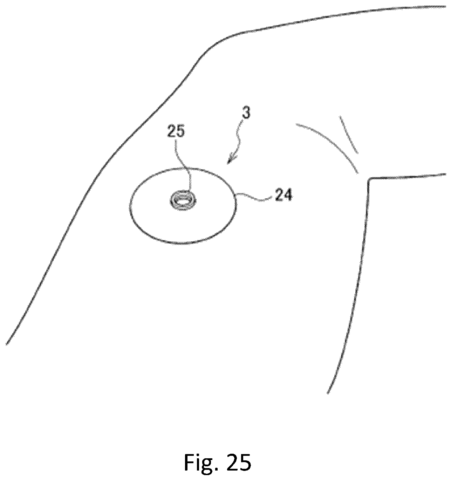

Figure 21 is a front view of a microneedle patch 3. The microneedle patch 3 is a combination of the sticky tape 24 and the microneedle base 25, with a microneedle array being provided on the microneedle base 25. Figure 22 is a right side view of the microneedle patch 3. Figure 23 is plan view of the microneedle patch 3. Figure 24 is a bottom view of the microneedle patch 3. Figure 25 is a perspective view showing a state in which the microneedle patch 3 is attached to an arm.

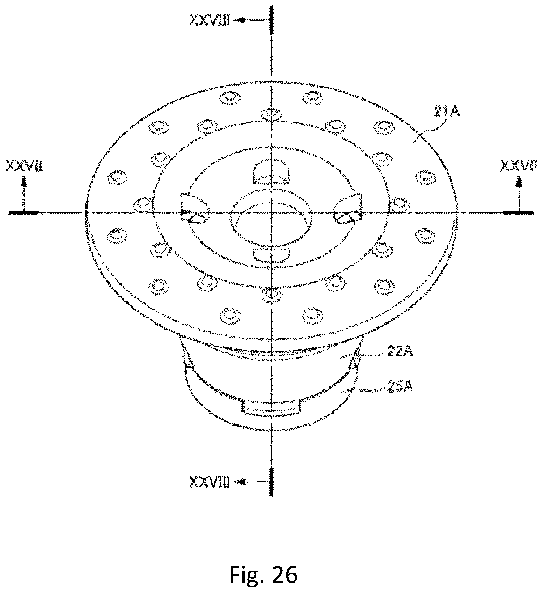

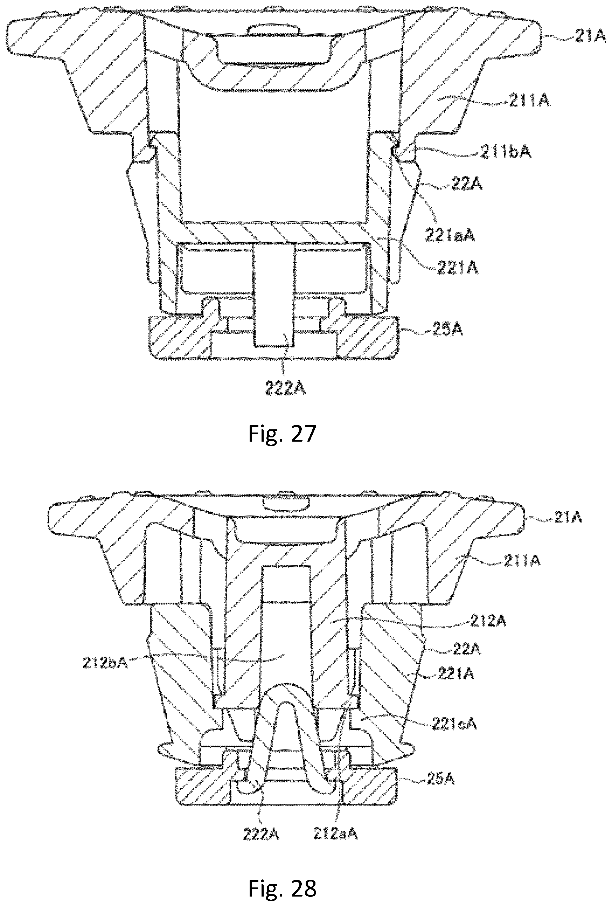

Next, a pusher 21A, a holder 22A, and a microneedle base 25A, which are shown in Figure 26, will be described. The pusher 21A, the holder 22A, and the microneedle base 25A are variations of the pusher 21, the holder 22, and the microneedle base 25. Figure 27 is a sectional view showing a section taken along line XXVII-XXVII in Figure 26. Figure 28 is a sectional view showing a section taken along line XXVIII-XXVIII in Figure 26.

As shown in Figure 27, the pusher 21A includes a button 211A. The holder 22A includes a central recess 221A and a hook portion 222A. The central recess 221A includes a temporary holding protrusion 221aA. A button 211A of the pusher 21A has been fitted over the central recess 221A from above, covering an outer circumference of the central recess 221A. The button 211A includes a temporary holding protrusion 211bA. A temporary holding protrusion 221aA is provided on an upper end of the central recess 221A of the holder 22A. The temporary holding protrusion 211bA of the button 211A and the temporary holding protrusion 221aA of the central recess 221A are fitted together. In a direction in which the pusher 21A and the holder 22A are spaced away from each other, the temporary holding protrusion 211bA and the temporary holding protrusion 221aA are fitted together, thereby restricting movements.

As shown in Figure 28, a central projection 212A is provided inside the button 211A of the pusher 21A. The central projection 212A is inserted in the central recess 221A. Indicator protrusions 221cA are provided in the central recess 221A. The indicator protrusions 221cA are provided protruding radially inward from the central recess 221A. Indicator protrusions 212aA are provided in the central projection 212A of the pusher 21A. The indicator protrusions 212aA are provided at positions corresponding to the indicator protrusions 221cA, protruding radially outward. When the user depresses the pusher 21A, the indicator protrusions 212aA run upon the indicator protrusions 221cA, eventually fitting under the indicator protrusions 221cA. In so doing, a notification sound is generated, notifying the user that the puncture is complete.

The central projection 212A of the pusher 21A is inserted in the central recess 221A from above. The central projection 212A includes a pushing groove 212bA. The pushing groove 212bA is abutted against the hook portion 222A. As described above, in the direction in which the pusher 21A and the holder 22A are spaced away from each other, the temporary holding protrusion 211bA and the temporary holding protrusion 221aA are fitted together, thereby restricting movements. In a direction in which the pusher 21A is pushed into the holder 22A, the pushing groove 212bA abuts the hook portion 222A, thereby restricting movements.

The present embodiment has been described above with reference to concrete examples. However, the present disclosure is not limited to these concrete examples. When those skilled in the art make design changes to any of the concrete examples as appropriate, the resulting variations are also included in the scope of the present disclosure as long as the variations contain features of the present disclosure. The components of the above-described concrete examples as well as the arrangements, conditions, shapes, and the like of the components are not limited to those illustrated above, and may be changed as appropriate. The components of the above-described concrete examples can be changed in combinations as appropriate as long as there is no technical contradiction.

## Claims

1. An applicator for puncturing skin with a microneedle array, the applicator comprising:
a holder configured to hold a microneedle base on which the microneedle array is provided;
a pusher configured to push the holder toward the skin; and
a shell that includes a first part to contact with the skin and a second part configured to hold the holder, the shell being deformable while maintaining an orientation of the microneedle base by connecting the first part and the second part.

2. The applicator according to claim 1,
wherein the pusher is provided so as to be slidable relative to the holder,
the holder includes a hook portion configured to hold the microneedle base, and
when the pusher slides relative to the holder, the hook portion releases the microneedle base.

3. The applicator according to claim 2,
wherein the hook portion deforms under an external force applied via the pusher, and thereby releases the microneedle base.

4. The applicator according to claim 3,
wherein an opening is provided in the microneedle base, and
the hook portion is placed extending outward from inside the opening, thereby holding the microneedle base.

5. The applicator according to claim 4,
wherein, as a projection provided on the microneedle base enters a recess provided in the holder, the microneedle base is positioned with respect to the holder, and
the opening is provided inside the projection.

6. The applicator according to claim 2, comprising:
an indicator configured to slide the pusher relative to the holder and generate a notification sound when a pushing force toward the skin exceeds a predetermined value.

7. The applicator according to claim 6,
wherein the indicator generates the notification sound through rubbing between the holder and the pusher.

8. The applicator according to claim 7,
wherein the indicator is provided to include a plurality of indicators.

9. The applicator according to claim 8,
wherein when the pusher slides relative to the holder, notification sounds generated by the plurality of indicators are synchronized.

10. The applicator according to claim 1,
wherein ridges and valleys configured to deflect while maintaining an orientation of the microneedle base when the pusher pushes the holder toward the skin are radially provided alternately on the shell.

11. The applicator according to claim 1 or 10,
wherein, as the holder is passed through a through-hole provided in the shell, the shell holds the holder,
the shell includes a side wall that forms the through-hole, and
the holder includes a return part protruding toward the side wall.

12. The applicator according to claim 11,
wherein the side wall includes a bend that conforms in shape to the return part, and
the holder includes a rising part that rises from a base end of the return part toward an inner edge of the through-hole by expanding in diameter.

13. A microneedle patch kit, comprising:
the applicator according to any one of claims 1 to 12, the microneedle base, an adhesive layer formed on a flange of the shell, and a protective sheet configured to close an opening in the shell by abutting the adhesive layer.

14. A microneedle patch, comprising:
a microneedle base on which a microneedle array for puncturing skin is provided; and
a sticky tape for fixing the microneedle base to the skin.
